# EUROPEAN PATENT APPLICATION

(11) **EP 4 659 678 A1**
(43) Date of publication of application: **10.12.2025**
(21) Application number: 24180232.1
(22) Date of filing: 05.06.2024
(51) Int. Cl.: A61B 6/03, A61B 6/00, G06T 7/00, H04N 19/172

(54) **SMART FRAME RATE ADAPTATION BY TRACKING BOLUS AGENT FLOW IN FLUOROSCOPY**

(71) Applicant: Siemens Healthineers AG, 91301 Forchheim (DE)
(72) Inventor: BISWAL, Mrutyunjay, 560100 Bangalore (IN); GNANAMANI, Murali Tharan, 560100 Bangalore (IN); HUSSAIN B., Tasheer, 560100 Bangalore (IN); MISHRA, Vivek, 560103 Bangalore (IN)
(74) Representative: HKW Intellectual Property PartG mbB

(57) **Abstract**

The present invention relates to a system for smart frame rate adaptation by tracking bolus agent flow in fluoroscopy, a method for using said system, and a computer program product.

A system for acquiring a number of medical images of a patient is suggested. The system comprises: a medical imaging unit for acquiring the number of medical images with a certain value of a frame rate; an image analysis unit for analyzing the acquired number of medical images; an event detection unit for detecting at least one event based on the analyzing of the acquired number of medical images; and a frame rate determination unit for determining a modified value of the frame rate based on the detected at least one event and the certain value of the frame rate.

The system can minimize the radiation exposure of the patient.

## Description

The present invention relates to a system for smart frame rate adaptation by tracking bolus agent flow in fluoroscopy, a method for using said system, and a computer program product.

Fluoroscopy is an imaging technic that uses X-rays to obtain real-time moving images of the interior of an object, for example a human or animal patient. In the following, the object is referred to as patient. Independent of the grammatical term usage, individuals with male, female or other gender identities are included within the term. Furthermore, the hereby obtained images are referred to as medical images. The temporal rate at which the medical images are captured is referred to as frame rate. A higher value of the frame rate means that more medical images are captured per second, allowing for a smoother visualization of moving structures within the patient by avoiding spatial and/or temporal aliasing factors. However, the higher value requires a higher radiation dose and intensity, leading to an increased radiation exposure of the patient as well as of the operator. Moreover, capturing more frames per second may also lead to an increased computational effort and complexity of the system. Therefore, for fluoroscopy examinations, it is important to balance the frame rate respecting potential risks of increased radiation exposure as well as the requirement for a smooth visualization of moving structures.

According to internal knowledge of the applicant, the frame rate can be manually adapted by entering fixed frame rates for different fixed time spans during the examination of the patient, whereby said time spans are set prior to the examination. Nonetheless, in order to not miss the capturing of the moving structure of interest, the time spans with a higher frame rate have to be set conservatively, resulting in a longer duration of a higher frame rate, causing an unnecessarily increased radiation exposure of the patient.

It is one object of the present invention to provide an improved acquiring of medical images.

Accordingly, a system for acquiring a number of medical images of a patient is suggested. The system comprises:
a medical imaging unit for acquiring the number of medical images with a certain value of a frame rate;
an image analysis unit for analyzing the acquired number of medical images;
an event detection unit for detecting at least one event based on the analyzing of the acquired number of medical images; and
a frame rate determination unit for determining a modified value of the frame rate based on the detected at least one event and the certain value of the frame rate.

The system can be a fluoroscopy system or any other system capable of taking real-time moving medical images.

The medical imaging unit can be, for example an X-ray scanner, a mammography scanner, a computed tomography "CT" scanner or the like. Hereby, the patient can be positioned on a bed of the medical imaging unit, be positioned on a chair, standing, or the like. The medical imaging unit may comprise an X-ray source, shooting narrows beams of X-rays through the body of the patient, and a detector device, receiving the X-rays after transmitting the patient. Both the X-ray source and the detector may be located within one device, for example a gantry through which the bed may be moved, or be configured as two separate devices. The medical imaging unit may use special digital X-ray detectors that are located directly opposite of the X-ray source. As the X-rays leave the patient, they are picked up by the detectors and transmitted to a computer that processes the data to form the number of medical images. Hereby, the medical imaging unit is configured to acquire the number of medical images with the certain value of the frame rate.

The acquired number of medical images can be analyzed by the image analysis unit. In this context, the term analyzing can be understood as applying a model on the data of the number of medical images. Therefore, preferably, digital detectors are applied, easing said analysis. In case analog detectors are used in order to detect the X-ray beams, the data of the analog detectors can be translated into digital data before the analysis. Eventually, the image analysis unit is configured to provide a value presenting the content of each of the number of medical images.

The event detection unit is configured to detect at least one event based on the analyzing of the acquired number of medical images, in particular, based on the provided values representing the content of the number of medical images. The at least one event can coincide with an event occurring within the patient, for example the at least one event can coincide with the beginning of a movement of a structure within the patient. Preferably, the event detection unit is configured to detect a plurality of events. The plurality of events can coincide with the beginning and the end of a movement occurring in the patient. Furthermore, the plurality of events can be allocated to a plurality of movements within the patient.

Based on the detected at least one event and the certain value of the frame rate, the frame rate determination unit is configured to determine the modified value of the frame rate. The modified value of the frame rate may differ from the certain value of the frame rate. In particular, the medical imaging unit can be configured to take a subject of the number of medical images with the certain value of the frame rate, which are then used for the analyzing and the detecting of the at least one event. With the detection of the at least one event, the frame rate determination is configured to determine the modified value of the frame rate. Consecutively, a following number of the number of medical images can be acquired with the modified value of the frame rate. In consequence, the suggested system is capable of automatically adapting the frame rate with which the medical imaging unit is acquiring the number of medical images by automatically analyzing the acquired number of medical images during the acquiring of the number of medical images. Hereby, the system can automatically adapt the frame rate at the occurring of the at least one event, for example, the movement of the structure of interest within the patient.

In a beneficial manner, the suggested system enables the acquisition of real-time medical images with varying frame rates while at the same time rendering unnecessary the definition of fixed time spans correlating to the varying frame rates prior to the acquiring of the number of medical images. Instead, a variation of the frame rate can be triggered by the at least event during the examination. Thus, the time spans of a higher frame rate can be minimized and, hereby, the radiation exposure of the patient be minimized.

According to an embodiment, the at least one event is an entry of a bolus within the acquired number of medical images or the exit of the bolus from the acquired number of medical images.

The structure of interest within the patient can comprise the bolus. The bolus can be a discrete amount of medication, drug, contrast agent, or the like. The bolus can be characterized by a spatially limited extension, possibly while moving through the body of the patient. The bolus can be used to visualize specific parts and/or components of the body of the patient. The bolus can be swallowed through the mouth of the patient and/or injected in an intravenous manner. Other ways of inserting the bolus into the body of the patient are feasible as well. The entry of the bolus within the acquired number of medical images can be understood as the entry of the bolus within that part of the patient's body, which is captured within the acquired number of medical images. In an analog way, the exit of the bolus from the acquired number of medical images can be understood as the exiting of the bolus from that part of the patient's body which is captured within the acquired number of medical images.

According to a further embodiment, the entry and/or the exit of the bolus is referred to boundaries of a limited field of interest within the acquired number of medical images.

The limited field of interest can be an image section within each of the acquired number of medical images. The field of interest can also be referred to as a subframe. The boundaries of the field of interest separate the field of interest from the rest of the respective one of the acquired number of medical images. For example, the field of interest can cover a specific part of the patient's body, for example a specific organ or organ part. Hence, the entry and/or exit of the bolus can be defined as the crossing of the boundaries within the number of the medical images by the bolus. By specifying the limited field of interest, the time duration of a higher frame rate can be minimized to the duration during which the movement of the bolus is occurring within the field of interest. Hence, said time duration and, accordingly, the radiation exposure of the patient, can be pinpointed and minimized.

According to a further embodiment, the field of interest is defined prior to the acquiring of the medical images.

The definition of the field of interest can be understood as defining the locations of the boundaries of the field of interest. Preferably, the field of interest is defined prior to the acquiring of the number of medical images. Hereby, the field of interest can be set according to a specific purpose of the examination. For example, in the frame of a diagnosis of the lung of the patient, the boundaries of the field of interest can be set around the lung of the patient as visible in the number of medical images. Alternatively, the field of interest can also be defined during the acquiring of the number of medical images, for example, dynamically. It is feasible, that the field of interest at least partly follows the movement of the bolus within the patient. In a beneficial manner, the field of interest can thus be defined according to the purpose of the current examination.

According to a further embodiment, the image analysis unit is configured for calculating an entropy-based metric of the acquired number of medical images.

In information theory, the entropy is a metric to measure a change of information. In the present context, the entropy-based metric can be used to measure the change of information within the number of medical images. In particular, the entropy-based metric can be used to measure the change of information between two subsequent ones of the number of medical images. In other words, the entropy-based metric can be used to quantify how much a specific one of the number of medical images differs from the respective previous one. Any movement occurring during the acquisition of specific ones of the number of medical images results in a high value of the entropy-based metric of the respective medical images. On the other hand, an absence of a movement during the acquisition of the number of medical images results in subsequent ones of the medical images showing substantially a similar content, and, thus, a low value of the entropy-based metric.

According to a further embodiment, the event detection unit is configured to detect the at least one event based on a variation of the calculated entropy-based metric. In particular, the event detection unit is configured to determine a time stamp of the detected at least one event.

The event detection unit can be configured to load the entropy-based metric calculated by the image analysis in order to detect the at least one event. The variation of the calculated entropy-based metric can be, for example, a change from a low value of the entropy-based metric to a high value of the entropy-based metric or vice versa. The at least one event can be characterized by the beginning and/or the end of a movement within the acquired number of medical images, thus, characterized by the beginning and/or the end of significant change of information within the acquired number of medical images. Said beginning and/or end of the change of information can result in a variation of the entropy-based metric, for example, the beginning and/or end of a peak value of the entropy-based metric. With the detection of the at least one event, the event detection unit can provide the respective time stamp at which the respective detected at least one event is occurring. In a beneficial manner, the time stamp of the detected at least one event can be detected automatically during the acquisition of the number of medical images.

According to a further embodiment, the event detection unit is configured to detect the at least one event based on a similar variation of the calculated entropy-based metric during subsequent ones of the acquired number of medical images.

In this context, the similar variation can be understood as a variation of the entropy-based metric with an identical sign and/or of a comparable magnitude occurring during a number of subsequent ones of the number of medical images. In other words, the similar variation can be understood as a variation of the entropy-based metric during a number of subsequent ones of the acquired number of medical images "aiming towards the same direction". A buffer window can be defined as the specific number of subsequent medical images. For example, the buffer window can be defined as three subsequent medical images. The event detection unit can be configured to detect the at least one event in case all the medical images acquired within the buffer window show a similar variation of the entropy-based metric, hence, a variation concerning the same sign and/or of a comparable magnitude. This way, significant changes of information within the number of medical images can be distinguished from minor changes of information due to for example breathing of the patient and/or signal noise or the like. In consequence, a false positive detection of the at least one event can be avoided.

According to a further embodiment, the entropy-based metric is a weighted combination of Forward and Backward Kullback Leibler Divergence.

The entropy-based metric can be a Forward Kullback Leibler Divergence or a Backward Kullback Leibler Divergence. The Backward Kullback Leibler Divergence can also be referred to as reverse Kullback Leibler Divergence. Nonetheless, it is preferred to use a combined Forward and Backward Kullback Leibler Divergence wherein both components are weighted. Said combination is also known as relative entropy.

According to a further embodiment, the event detection unit is configured to define an action time window between two determined time stamps corresponding to two detected events.

The two determined time stamps corresponding to two detected events can represent the time stamp of the entry of the bolus and the time stamp of the exit of the bolus. The time between said determined time stamps is hereby referred to as the action time window. In case both detected events are separated from one another by a significant time duration, for example, due to a large extension of the bolus, the two events can be characterized by two separate peak values of the entropy-based metric. In this case, the action time window can be set as the time duration between both peaks. In case both detected events are occurring shortly after one another, the entropy-based metric can show only one longer peak value. In this case, the two events can be determined as the beginning or the end, respectively, of the single peak. In consequence, the action time window can be defined as the duration of the single peak. The event detection unit can also be configured to define a plurality of action time windows, for example, due to a plurality of boluses inserted into the patient during a single examination.

According to a further embodiment, the frame rate determination unit is configured to increase the value of the frame rate during the defined action time window.

The frame rate determination unit can be configured to load the location or locations of the at least one action time window defined by the event detection unit. The frame rate determination unit can be configured to increase the modified value of the frame rate during the at least one action time window. Hence, during the time of the at least one action time window, the frame rate with which the respective number of medical images are acquired can be increased in comparison to the time outside of the at least one action time window. In particular, at the beginning of the at least one action time window, the value of the frame rate can be increased while at the end of the at least one action time window, the value of the frame rate can be reduced again. The increased value of the frame rate can be the modified value of the frame rate. Hence, the at least one action time window can be characterized as at least one time duration, during which the medical imaging unit is acquiring the respective number of medical images with a higher frame rate than outside of the at least one action time window.

According to a further embodiment, the frame rate comprises the values of a standby frame rate and an action frame rate, wherein the action frame rate is higher than the standby frame rate.

The frame rate can have multiple values. One value of the frame rate can be a standby frame rate. The standby frame rate can be the frame rate with which the medical imaging unit is acquiring medical images outside of the action time window. The frame rate can further comprise an action frame rate which can show a higher value than the standby frame rate. The action frame rate can be the frame rate with which the medical imaging unit is acquiring medical images during the action time window.

According to a further embodiment, the frame rate determination unit is configured to switch from the standby frame rate to the action frame rate with the beginning of the defined action time window and to switch from the action frame rate to the standby frame rate with the end of the defined action time window.

The beginning of the action time window corresponds to the time stamp of the entry event, caused by the entry of the bolus into one of the number of medical image or into the respective field of interest, respectively. In an analog way, the end of the action time window corresponds to the time stamp of the exit event, caused by the bolus exiting one of the number of medical images or the respective field of interest, respectively.

According to a further embodiment, the frame rate is limited by a minimum value and/or a maximum value.

In case the frame rate is set to a too low value, the risk of temporal and/or spatial aliasing factors is emerging, resulting in a reduced quality of the acquired number of medical images and, thus, threatening the precision of the diagnosis based on the acquired number of medical images. Therefore, the minimum value can be set as a lower limit for the frame rate. The minimum value can correspond to the standby frame rate. On the other hand, according to internal knowledge of the applicant, the radiation dose of the patient scales with the square root of the frame rate. Hence, in order to avoid overdosing, the maximum value can be set as an upper limit for the frame rate. The maximum value can correspond to the action frame rate. The minimum value and/or the maximum value can be defined prior to the acquiring of the number of medical images and pay respect to the purpose of the current examination and/or pay respect to the body parts captured within the number of medical images. In a beneficial manner, the frame rate can be set at an optimal value throughout the whole examination resulting in an overall radiation exposure of the patient which is as low as possible and as high as necessary.

The respective entities, e.g. the image analysis unit, the event detection unit, and/or the frame rate determination unit, may be implemented in hardware and/or in software. If said entity is implemented in hardware, it may be embodied as a de-vice, e.g. as a computer or as a processor or as a part of a system, e.g. a computer system. If said entity is implemented in software it may be embodied as a computer program product, as a function, as a routine, as a program code or as an executable object.

Any embodiment of the first aspect may be combined with any embodiment of the first aspect to obtain another embodiment of the first aspect.

According to a second aspect, a method for using a system for acquiring a number of medical images of a patient is suggested. In particular, the system is embodied according to the first aspect or any embodiment of the first aspect. The method comprises:
acquiring the number of medical images with a certain value of a frame rate by a medical imaging unit;
analyzing the acquired number of medical images by an image analysis unit;
detecting at least one event based on the analyzing of the acquired number of medical images by an event detection unit; and
determining a modified value of the frame rate based on the detected at least one event and the certain value of the frame rate by a frame rate determination unit.

According to a third aspect, a computer program product comprising instructions is proposed which, when the program is executed by a computer, cause the computer to carry out the above-mentioned method.

A computer program product, such as a computer pro-gram means, may be embodied as a memory card, USB stick, CD-ROM, DVD or as a file which may be downloaded from a server in a network. For example, such a file may be provided by transferring the file comprising the computer program product from a wireless communication network.

The embodiments and features described with reference to the system of the present invention apply mutatis mutandis to the method of the present invention.

Further possible implementations or alternative solutions of the invention also encompass combinations - that are not explicitly mentioned herein - of features described above or be-low with regard to the embodiments. The person skilled in the art may also add individual or isolated aspects and features to the most basic form of the invention.

Further embodiments, features and advantages of the present invention will become apparent from the subsequent description and dependent claims, taken in conjunction with the accompanying drawings, in which:
Fig. 1 shows a schematic perspective illustration of an embodiment of a system for acquiring a number of medical images of a patient with a value of a frame rate;
Fig. 2 shows a schematic illustration of an embodiment of one of the number of medical images acquired by the system according to Fig. 1 at a first time stamp;
Fig. 3 shows a schematic illustration of an embodiment of a further one of the number of medical images acquired by the system according to Fig. 1 at a second time stamp;
Fig. 4 shows a schematic illustration of an embodiment of a further one of the number of medical images acquired by the system according to Fig. 1 at a third time stamp;
Fig. 5 shows a schematic illustration of an embodiment of an entropy-based metric according to an analysis of the number of medical images acquired by the system according to Fig. 1;
Fig. 6 shows a schematic illustration of an embodiment of a temporal evolution of the value of the frame rate for acquiring the number of medical images with the system according to Fig. 1; and
Fig. 7 shows an embodiment of a method for using the system according to Fig. 1.

In the Figures, like reference numerals designate like or functionally equivalent elements, unless otherwise indicated.

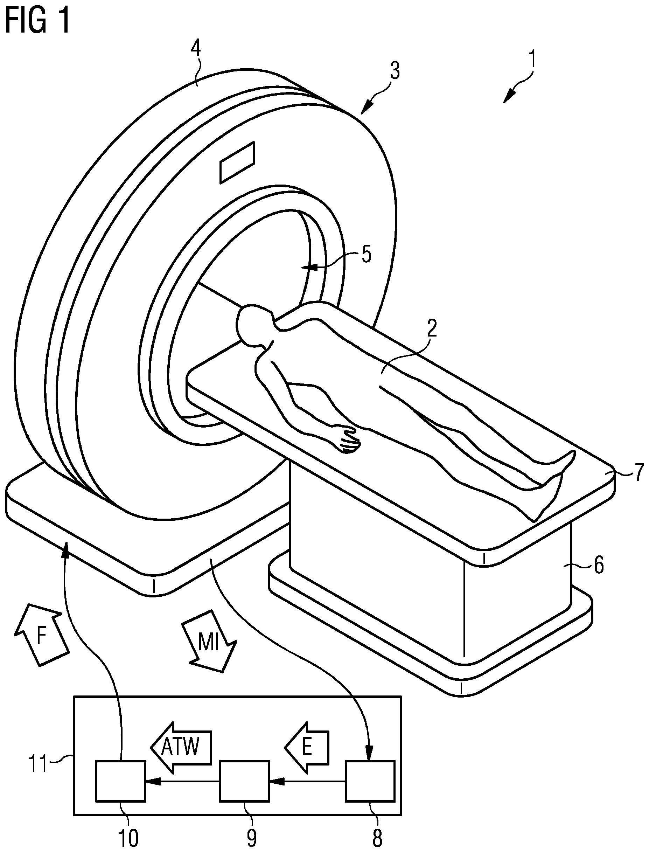

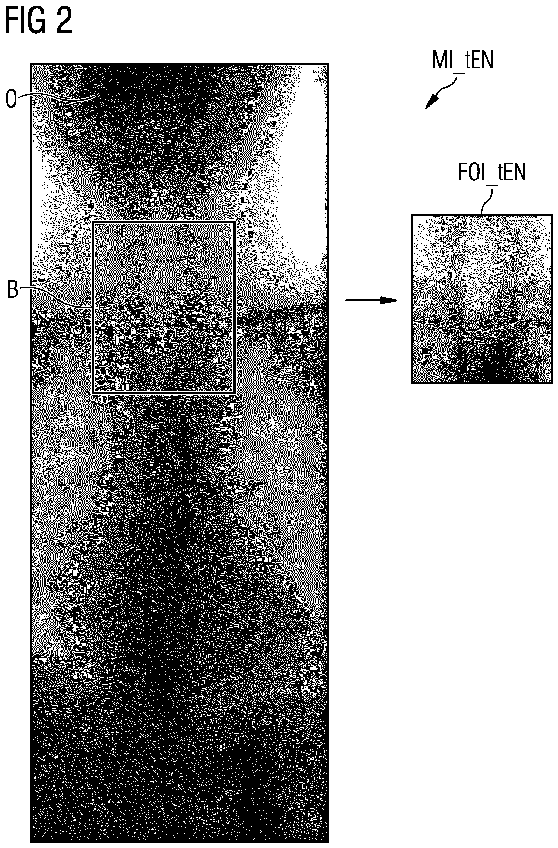

Fig. 1 shows a schematic perspective illustration of an embodiment of a system 1 which can be used in the frame of a fluoroscopy examination for acquiring a number of medical images MI (see Figs. 2 to 4) of a patient 2 with a frame rate F. It is an object of the fluoroscopy examination to capture a movement within the patient 2, in particular, the movement of a bolus O (see Figs. 2 to 4) within the patient 2. The bolus O can be a contrast agent and/or a medication which can be swallowed by the patient 2 and/or injected intravenously, wherein the bolus O can be limited in geometrical and/or temporal extension when moving through the patient 2. Hereby, a radiation exposure of the patient 2 due to the examination rises in square with the frame rate F. On the other hand, the quality and, hereby, the usability and reliability of the acquired number of medical images MI suffers from a too low value of the frame rate F due to temporal and/or spatial aliasing factors. The system 1 is capable of optimizing the frame rate F. In particular, the system 1 is configured to automatically adapt the frame rate F to higher values for a limited time span, wherein the limited time span corresponds to the occurring of the movement of the bolus O within a limited field of interest FOI (see Figs. 2 to 4), wherein the field of interest FOI represents an image section of the acquired medical images MI.

The system 1 comprises a medical imaging unit 3 with a gantry 4, whereby the gantry 4 contains an X-ray source and a digital X-ray detector (both not visible in Fig. 1). The gantry 4 surrounds a tube 5. The medical imaging unit 3 further comprises a table 6 onto which a bed 7 is attached. The bed 7 is attached to the table 6 such way that the bed 7 can be moved through the gantry 4. Note that other embodiments of the medical imaging unit 3 are feasible as well. For example, the X-ray source and the digital X-ray detector can be designed as two separate devices which may be positioned stationary, hence, not moving during the fluoroscopy examination. Furthermore, the patient 2 can be seated on a chair or be standing as well, wherein the patient 2 can be positioned between the X-ray source and the digital X-ray detector.

The digital X-ray detector is configured to acquire the number of medical images MI by detecting X-ray beams transmitting the patient 2. Hereby, the acquired number of medical images MI offer a view inside a body of the patient 2 with respect to a specific optical field of view of the medical imaging unit 3 (see Figs. 2 to 4). The field of view can be selected prior to the acquiring of the number of medical images MI. Exemplary ones of the acquired number of medical images MI can be schematically seen in Figs. 2 to 4.

The system 1 further comprises an image analysis unit 8 for analyzing the acquired number of medical images MI.

The system 1 further comprises an event detection unit 9. The event detection unit 9 is configured for detecting at least one event based on the analyzing of the acquired number of medical images MI. Preferably, the event detection unit 9 is configured for detecting a plurality of events based on the analyzing of the acquired number of medical images MI.

The system 1 further comprises a frame rate determination unit 10 for determining a modified value of the frame rate F based on the detected at least one event and on the certain used value of the frame rate F.

The image analysis unit 8, the event detection unit 9, and the frame rate determination unit 10 can be designed as separate computer products. They can be part of a control unit 11, which can be designed as an independent computer program product or can be designed as part of a computer program product for controlling the medical imaging unit 3. The control unit 11 can be executed on a local computer, on a server (both not shown in Fig. 1), or it can be executed within the medical imaging unit 3. The image analysis unit 8, the event detection unit 9, and the frame rate determination unit 10 will be described in detail below with reference to Figs. 2 to 5.

Eventually, the modified value of the frame rate F as determined by the frame rate determination unit 10 is provided to the medical imaging unit 3. In consequence, in the following, the medical imaging unit 3 is configured to acquire the number of medical images MI with the modified value of the frame rate F.

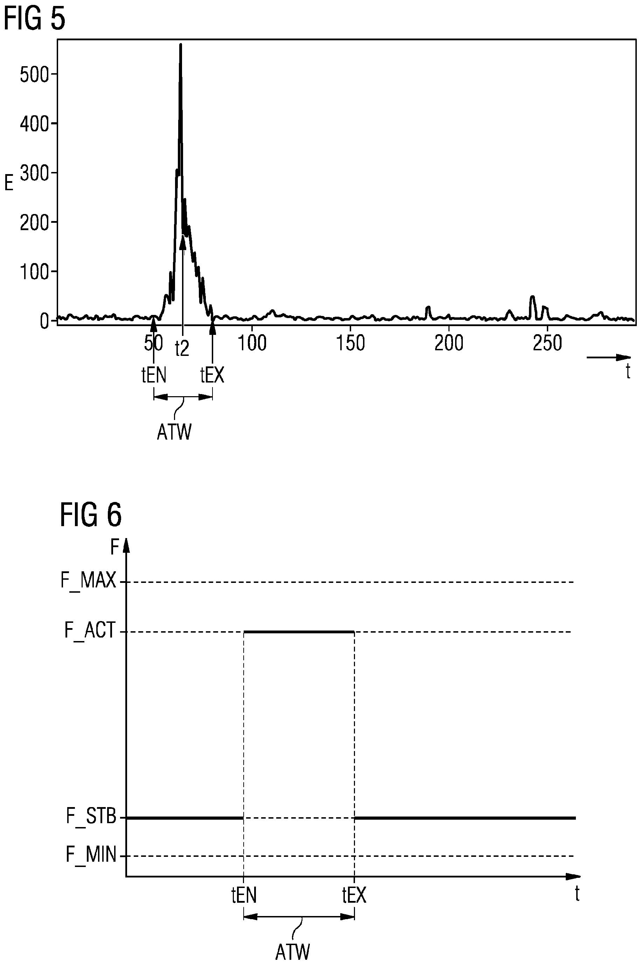

Figs. 2 to 4 show three schematic illustrations of exemplary embodiments of the acquired number of medical images MI and the respective field of interests FOI, acquired at three different time stamps during the examination by the above-mentioned system 1. Note, that the illustrated content of the shown ones of the acquired number of medical images MI and the respective field of interests FOI is not part of the invention but shown for illustrative purposes only. Fig. 5 shows a schematic illustration of an embodiment of an entropy-based metric E according to an analysis of the acquired number of medical images MI. In the following, Figs. 2 to 5 will be referred to at the same time.

The image analysis unit 8 is configured to load the acquired number of medical images MI from the medical imaging unit 3 into the image analysis unit 8 and to analyze the acquired number of medical images MI. The analysis of the acquired medical images MI can include the whole content of the acquired number of medical images MI. Nonetheless, in a preferred embodiment, the image analysis unit 8 is configured to analyze the respective limited field of interest FOI of each of the acquired number of medical images MI only. The field of interest FOI can be defined as an image section within a medical image MI, wherein the extent of the field of interest FOI can be set by a boundary B and can be defined prior to the acquiring the number of medical images MI. In this context, analyzing the acquired number of medical images MI can be understood as applying a metric for determining a change of information within a current one of the acquired number of medical images MI, in particular, within the respective field of interest FOI, in respect to a previous one of the acquired number of medical images MI. Hereby, the image analysis unit 8 is configured for calculating the entropy-based metric E, particularly a combined Forward and Backward Kullback-Leibler Divergence (KLD). The calculated entropy-based metric E according to the illustrated embodiment is illustrated over the time t of the examination in Fig. 5.

Fig. 2 shows an exemplary embodiment of one of the acquired number of medical images MI_tEN, acquired at the time stamp tEN. In addition, Fig. 2 shows the respective limited field of interest FOI_tEN. At the time stamp tEN of the shown exemplary embodiment, the bolus O is about to cross the boundary B. Note, that within the illustrated temporal evolution of the entropy-based metric E in Fig. 5, the time stamp tEN is marked on the horizontal axis. With the time stamp tEN, the magnitude of the entropy-based metric E significantly rises due to the entering of the bolus O into the field of interest FOI_tEN and the correlated change of information within the field of interest FOI_tEN.

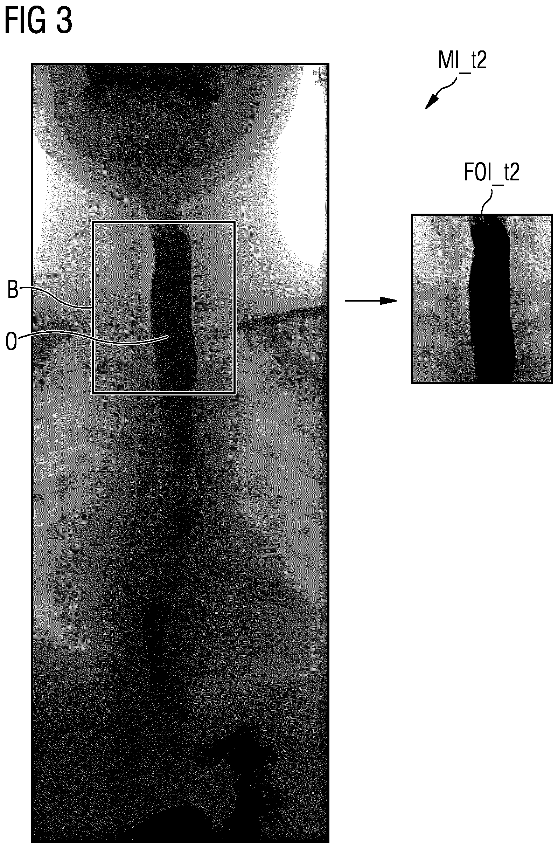

In Fig. 3, a further exemplary embodiment of one of the acquired number of medical images MI_t2 is illustrated, acquired at the time stamp t2. The time stamp t2 is located after the time stamp tEN and represents in an exemplary manner the bolus O being located substantially in the center of the respective limited field of interest FOI_t2. The motion of the bolus O through the field of interest FOI results in a significant change of information within the field of interest FOI of each of the acquired number of medical images MI which captures the bolus O within the field of interest FOI. This change of information results in maintaining a high value of the entropy-based metric E.

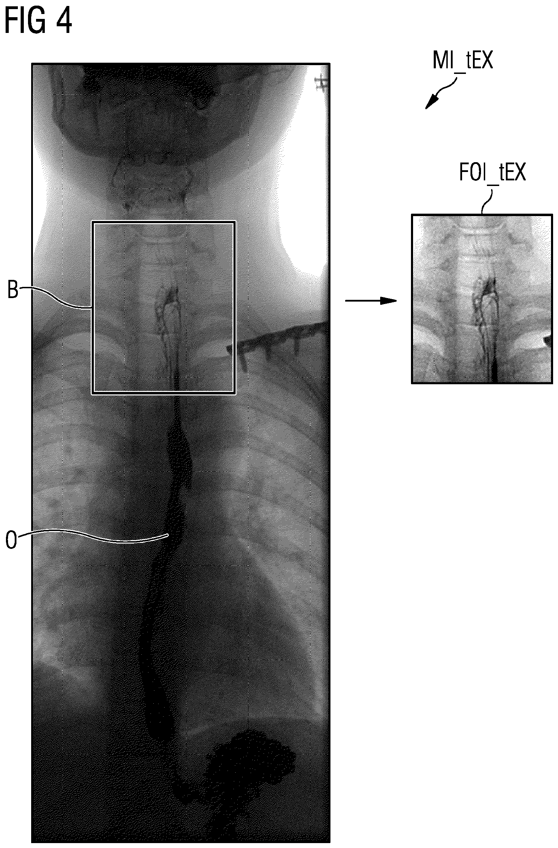

In Fig. 4, a further exemplary embodiment of one of the acquired number of medical images MI_tEX is illustrated acquired at the time stamp tEX. The time stamp tEX corresponds to the event of the bolus O exiting the field of interest FOI_tEX. Note, that in Fig. 4, the bolus O is visible within the medical image MK_tEX but outside of the field of interest FOI_tEX. With the exiting of the bolus O out of the field of interest FOI_tEX, the change of information within the field of interest FOI_tEX returns to a minor value and, hence, the peak value of the entropy-based metric E ends at the time stamp tEX, as illustrated in Fig. 5.

To sum up, the motion of the bolus O through the field of interest FOI is represented by the peak of the entropy-based metric E as illustrated in Fig. 5.

The event detection unit 9 is configured to detect the at least one event based on the entropy-based metric E, as derived by the image analysis unit 8. In particular, the at least one event can correspond to the bolus O entering and/or exiting the field of interest FOI, hence, the bolus O crossing the boundary B. In consequence, based on the calculated entropy-based metric E, the event detection unit 9 is configured to detect the bolus O entering and/or the exiting the field of interest FOI.

The event detection unit 9 is configured to detect the at least one event based on a significant variation of the entropy-based metric E. In the exemplary embodiment shown in Fig. 5, the at least one event corresponds to the beginning or to the end, respectively, of the peak value of the entropy-based metric E, occurring at the time stamps tEN or tEX, respectively. In order to distinguish the at least one event from minor variations or signal noise of the entropy-based metric E, the event detection unit 9 may be configured to detect the at least one event using a buffer window, wherein the buffer window is defined as a number of subsequent ones of the acquired number of medical images MI, during which the entropy-based metric E has to vary in the same direction and/or with a significant magnitude in order to detect the at least one event. The buffer window can be defined, for example, as three subsequent ones of the acquired number of medical images MI. In consequence, for example, the at least one event is detected in case the magnitude of the entropy-based metric E rises significantly during three subsequent ones of the acquired number of medical images. This way, a false positive detection of the at least one event can be avoided and/or prevented.

The event detection unit 9 is further configured to define an action time window ATW (see Fig. 5) between the time stamps tEN and tEX corresponding to two subsequent detected events. In consequence, the action time window ATW corresponds to the time duration during which the bolus O is moving through the field of interest FOI.

Fig. 6 shows a schematic illustration of an embodiment of a temporal evolution of the value of the frame rate F over time t for acquiring the number of medical images MI with the above-mentioned system 1. In other words, Fig. 6 illustrates, according to the exemplary embodiment of the calculated entropy-based metric E shown in Fig. 5, with which value of the frame rate F the number of medical images MI are acquired at each of the time stamps throughout the time t of the examination.

The frame rate determination unit 10 is configured to initially set the frame rate F for acquiring the number of medical images MI to a certain value, which value can be referred to as a standby frame rate F_STB. The standby frame rate F_STB is set prior to the acquiring of the number of medical images MI and can correspond to or be above a minimum frame rate F_MIN. The minimum frame rate F_MIN can correspond to the lowest feasible value of the frame rate F in order to avoid spatial and/or temporal aliasing effects. The frame rate determination unit 10 is configured to maintain the value of the frame rate F at the certain value, for example, the standby frame rate F_STB, as long as the event detection unit 9 does not detect the at least one event.

With the detection of the at least one event at the time stamp tEN and the corresponding beginning of the action time window ATW, the frame rate determination unit 10 is configured to increase the value of the frame rate F from the certain value to a modified value being higher, whereby the modified value of the frame rate F can correspond to an action frame rate F_ACT. The value of the action frame rate F_ACT can correspond to or be lower as a maximum frame rate F_MAX which is set prior to the acquiring of the number of medical images MI. The maximum frame rate F_MAX can correspond to the highest feasible value of the frame rate F concerning radiation exposure of the patient 2 and/or technical limitations of the medical imaging unit 3. The frame rate determination unit 10 is configured to transmit the determined modified value of the frame rate F to the medical imaging unit 3. In consequence, within the action time window ATW, the number of medical images MI are acquired by the medical imaging unit 3 with the value of the action frame rate F_ACT of the frame rate F.

The frame rate determination unit 10 is further configured to reduce the value of the frame rate F at the time stamp tEX, corresponding to the end of the action time window ATW, back to the value of the standby frame rate F_STB. In consequence, before and after the action time window ATW, the medical imaging unit 3 is configured to acquire the number of medical images MI with the reduced value of the standby frame rate F_STB.

Note that during the examination and the acquisition of the number of medical images MI, a plurality of action time windows ATW can occur. Furthermore, for example in case the bolus O is of a large geometrical and/or temporal extension, and, thus, the event of the bolus O entering the field of interest FOI and the event of the bolus O exiting the field of interest FOI are substantially separated from one another, the derived temporal evolution of the entropy-based metric E can show two distinct peaks, representing the entering or the exiting, respectively. In this case, the action time window ATW can be determined by the event detection unit 9 as the time duration between the two distinct peaks of the entropy-based metric E.

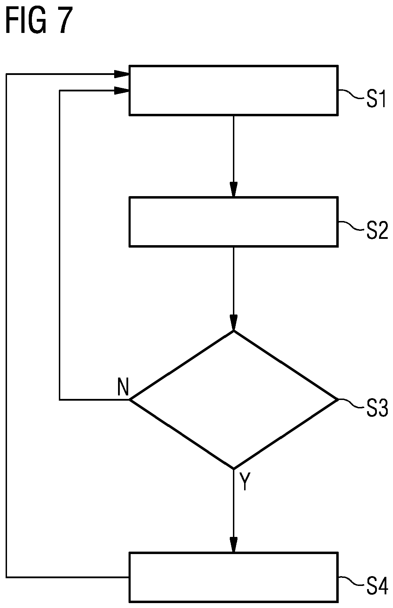

Fig. 7 shows an embodiment of a method for using the above-mentioned system 1.

In a first step S1, the number of medical images MI is acquired by the medical imaging unit 3 with the certain value of the frame rate F, wherein the certain value can correspond to the standby frame rate F_STB or the action fame rate F_ACT. In a second step S2, the acquired number of medical images MI are analyzed by the image analysis unit 8. In particular, the change of information within the acquired number of medical images MI is determined using the entropy-based metric E. In a third step S3, the event detection unit 9 detects the at least one event based on the analyzed acquired number of medical images MI. Hereby, the event detection unit 9 detects a variation of the entropy-based metric E. In case the event detection unit 9 detects the at least one event, a fourth step S4 is executed, wherein the modified value of the frame rate F is determined by the frame rate determination unit 10. In case the certain value of the frame rate F corresponds to the standby frame rate F_STB, the modified value of the frame rate F corresponds to the action fame rate F_ACT. On the other hand, in case the certain value of the frame rate F corresponds to the action frame rate F_ACT, the modified value of the frame rate F corresponds to the standby frame rate F_STB. Nonetheless, in case the event detection unit 9 does not detect the at least one event, it is proceeded with step S1, and the acquiring of the number of medical images MI is continued with the certain value of the frame rate F.

Although the present invention has been described in accordance with preferred embodiments, it is obvious for the person skilled in the art that modifications are possible in all embodiments.

## Claims

1. A system (1) for acquiring a number of medical images (MI) of a patient (2), the system (1) comprising:
a medical imaging unit (3) for acquiring the number of medical images (MI) with a certain value of a frame rate (F);
an image analysis unit (8) for analyzing the acquired number of medical images (MI);
an event detection unit (9) for detecting at least one event based on the analyzing of the acquired number of medical images (Ml); and
a frame rate determination unit (10) for determining a modified value of the frame rate (F) based on the detected at least one event and the certain value of the frame rate (F).

2. The system according to claim 1, wherein the at least one event is an entry of a bolus (O) within the acquired number of medical images (MI) or the exit of the bolus (O) from the acquired number of medical images (MI).

3. The system according to claim 2, wherein the entry and/or the exit of the bolus (O) is referred to boundaries (B) of a limited field of interest (FOI) within the acquired number medical images (MI).

4. The system according to claim 3, wherein the field of interest (FOI) is defined prior to the acquiring of the number of medical images (MI).

5. The system according to one of claims 1 to 4, wherein the image analysis unit (8) is configured for calculating an entropy-based metric (E) of the acquired number of medical images (MI).

6. The system according to claim 5, wherein the event detection unit (9) is configured to detect the at least one event based on a variation of the calculated entropy-based metric (E), in particular, the event detection unit (9) is configured to determine a time stamp of the detected at least one event.

7. The system according to claim 6, wherein the event detection unit (9) is configured to detect the at least one event based on a similar variation of the calculated entropy-based metric (E) during subsequent ones of the acquired number of medical images (MI).

8. The system according to one of claims 5 to 7, wherein the entropy-based metric (E) is a weighted combination of Forward and Backward Kullback Leibler Divergence.

9. The system according to one of claims 6 to 8, wherein the event detection unit (9) is configured to define an action time window (ATW) between two determined time stamps (tEN, tEX) corresponding to two detected events.

10. The system according to claim 9, wherein the frame rate determination unit (10) is configured to increase the value of the frame rate (F) during the defined action time window (ATW).

11. The system according to claim 9 or 10, wherein the frame rate (F) comprises the values of a standby frame rate (F_STB) and an action frame rate (F_ACT), wherein the action frame rate (F_ACT) is higher than the standby frame rate (F_STB).

12. The system according to claim 11, wherein the frame rate determination unit (10) is configured to switch from the standby frame rate (F_STB) to the action frame rate (F_ACT) with the beginning (tEN) of the defined action time window (ATW) and to switch from the action frame rate (F_ACT) to the standby frame rate (F_STB) with the end (tEX) of the defined action time window (ATW).

13. The system according to one of claims 1 to 12, wherein the frame rate (F) is limited by a minimum value (F_MIN) and/or a maximum value (F_MAX).

14. A method for using a system (1), particularly according to one of claims 1 to 13, for acquiring a number of medical images (MI) of a patient (2), the method comprising:
acquiring (S1) the number of medical images (MI) with a certain value of a frame rate (F) by a medical imaging unit (3);
analyzing (S2) the acquired number of medical images (MI) by an image analysis unit (8);
detecting (S3) at least one event based on the analyzing of the acquired number of medical images (MI) by an event detection unit (9); and
determining (S4) a modified value of the frame rate (F) based on the detected at least one event and the certain value of the frame rate (F) by a frame rate determination unit (10).

15. A computer program product comprising instructions which, when the program is executed by a computer, cause the computer to carry out the method of claim 14.
